# EUROPÄISCHE PATENTSCHRIFT

(11) **EP 3 029 044 B1**
(45) Veröffentlichungstag und Bekanntmachung des Hinweises auf die Patenterteilung: **01.05.2019**
(21) Anmeldenummer: 14196178.9
(22) Anmeldetag: 04.12.2014
(51) Int. Cl.: C07F 9/6574, C07B 41/06, C07C 45/50

(54) **BISPHOSPHITE DIE EINEN UNSYMMETRISCHEN BIARYL-ZENTRAL-BAUSTEIN AUFWEISEN**
BIS-PHOSPHITES WITH AN ASYMMETRIC BIARYL CENTRAL COMPONENT
BISPHOSPHITES PRÉSENTANT UN COMPOSANT CENTRAL EN BI-ARYLE ASYMÉTRIQUE

(43) Veröffentlichungstag der Anmeldung: 08.06.2016
(60) Teilanmeldung: 15173827.5
(73) Patentinhaber: Evonik Degussa GmbH, 45128 Essen (DE)
(72) Erfinder: Dyballa, Katrin Marie, 45657 Recklinghausen (DE); Franke, Robert, 45772 Marl (DE); Fridag, Dirk, 45721 Haltern am See (DE); Börner, Armin, 18059 Rostock (DE); Selent, Detlef, 18059 Rostock (DE)
(74) Vertreter: Evonik Patent Association

(56) Entgegenhaltungen:
- EP-A1- 3 029 046
- EP-B1- 0 900 187
- EP-B1- 3 029 048
- WO-A2-99/06146
- US-A- 5 874 641

## Beschreibung

Die Erfindung betrifft Bisphosphite, die einen unsymmetrischen Biaryl-Zentral-Baustein aufweisen. Des Weiteren deren Verwendung als Liganden in der Hydroformylierung.

Ein Bisphosphit weist einen Zentral-Baustein, das so genannte Rückgrat, und zwei Flügel-Bausteine auf, welche mit dem Zentral-Baustein über das P-Atom verbunden sind.

Die erfindungsgemäßen Biaryl-Zentral-Bausteine weisen beispielsweise eine Phenyl-Phenyl-Einheit, oder eine Naphthyl-Phenyl-Einheit auf.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigen Phosphor P^{III}. Eine gute Übersicht über den Stand der Hydroformylierung von Olefinen findet sich in B. CORNILS, W. A. HERRMANN, "Applied Homogeneous Catalysis with Organometallic Compounds", Vol. 1 & 2, VCH, Weinheim, New York, 1996 bzw. R. Franke, D. Selent, A. Börner, "Applied Hydroformylation", Chem. Rev., 2012, DOI:10.1021/cr3001803.

Jede katalytisch aktive Zusammensetzung hat ihre spezifischen Vorzüge. Je nach Einsatzstoff und Zielprodukt kommen daher unterschiedliche katalytisch aktive Zusammensetzungen zum Einsatz.

Die Patente US 4 694 109 und US 4 879 416 beschreiben Bisphosphinliganden und ihren Einsatz in der Hydroformylierung von Olefinen bei niedrigen Synthesegasdrücken. Besonders bei der Hydroformylierung von Propen werden mit Liganden dieses Typs hohe Aktivitäten und hohe n/i-Selektivitäten (n/i = das Verhältnis von linearem Aldehyd (=n) zu verzweigtem (=iso) Aldehyd)) erreicht. In WO 95/30680 werden zweizähnige Phosphinliganden und ihr Einsatz in der Katalyse, unter anderem auch in Hydroformylierungsreaktionen, offenbart.

In DE 10 2006 058 682 A1 werden Bisphosphite offenbart, welche einen symmetrischen Zentral-Baustein (Y) aufweisen. Dieser kann aus den in DE 10 2006 058 682 A1 Absatz [0022] offenbarten Formeln IIa bis IId oder III ausgewählt sein.

In EP 0 900 187 B1 wird ein Verfahren zur Herstellung von Hydroxyaldehyden beschrieben. Die Alkadiene werden hierbei einer reduzierenden Hydroformylierung mit anschließender Hydroformulierung unterzogen.

In WO 99/06146 werden Bisphosphitliganden beschrieben, welche zur Katalyse von Hydrocyanierungen von ungesättigten organischen Verbindungen verwendet werden können.

In US 5,874,641 wird ein Verfahren zur Herstellung von terminalen Aldehyden beschrieben. Hierbei kommt ein Rhodium-Katalysator mit bidentaten Phosphitliganden zum Einsatz.

Obgleich eine Vielzahl von Liganden und ihre Verwendung in der Rhodium-katalysierten Hydroformylierung bekannt sind, ist es wünschenswert, neue Liganden mit verbesserten Eigenschaften zu entwickeln.

Der Erfindung lag die Aufgabe zugrunde, Bisphosphite bereitzustellen, welche gegenüber den bekannten Bisphosphiten vorteilhafte Eigenschaften in der Hydroformylierung aufweisen. Insbesondere bestand die Aufgabe darin, neue Liganden bereitzustellen, die neben einer guten Ausbeute auch noch eine hohe n-Selektivität der korrespondierende Aldehyde bei der Umsetzung von endständigen Olefinen generieren und die ebenfalls bei der Hydroformylierung von innenständigen Olefinen zufriedenstellende n/i-Selektivitäten aufweisen. Es soll also neben einer guten Ausbeute auch noch zusätzlich eine gute Selektivität erzielt werden.

Die Aufgabe wird gelöst durch eine Verbindung nach Anspruch 1.

Verbindung, welche eine der folgenden Strukturen aufweist:

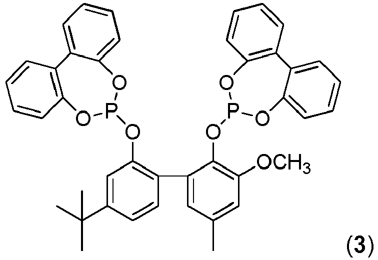

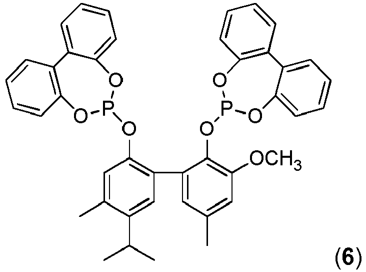

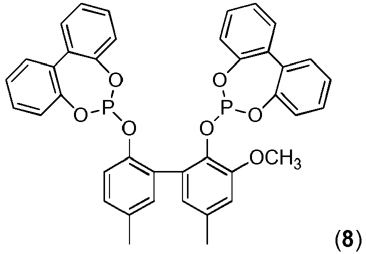

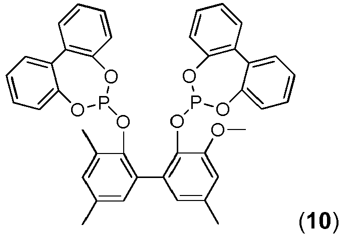

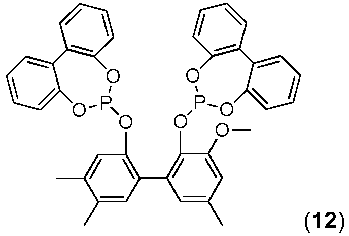

Neben den Verbindungen wird auch ein Komplex beansprucht, welcher diese Verbindungen umfasst.

Komplex umfassend:
- eine zuvor beschriebene Verbindung,
- ein Metallatom ausgewählt aus: Rh, Ru, Co, Ir.

In einer bevorzugten Ausführungsform ist das Metall Rh.

Siehe hierzu R. Franke, D. Selent, A. Börner, "Applied Hydroformylation", Chem. Rev., 2012, DOI:10.1021/cr3001803; S. 5688 Schema 12 "General Method for the Preparation of a P-Modified Rh precatalyst" und darin zitierte Literaturstellen sowie P. W. N. M. van Leeuwen, in Rhodium Catalyzed Hydroformylation, P. W. N. M. van Leeuwen, C. Claver (Hrsg.), Kluwer, Dordrecht, 2000 unter anderem S. 48 ff, S.233 ff. und darin zitierte Literaturstellen sowie K.D. Wiese und D. Obst in Top. Organomet. Chem. 2006, 18, 1-13; Springer Verlag Berlin Heidelberg 2006 S. 6 ff sowie darin zitierte Literaturstellen.

Des Weiteren wird die Verwendung der Verbindung als Ligand in einem Ligand-Metall-Komplex zur Katalyse einer Hydroformylierungsreaktion beansprucht.

Verwendung einer zuvor beschriebenen Verbindung in einem Ligand-Metall-Komplex zur Katalyse einer Hydroformylierungsreaktion.

Das Verfahren bei dem die Verbindung als Ligand in einem Ligand-Metall-Komplex zur Umsetzung eines Olefins zu einem Aldehyd eingesetzt wird, wird ebenfalls beansprucht.

Verfahren umfassend die Verfahrensschritte:
a) Vorlegen eines Olefins,
b) Zugabe eines zuvor beschriebenen Komplexes,
   oder einer zuvor beschriebenen Verbindung und einer Substanz, welche ein Metallatom ausgewählt aus: Rh, Ru, Co, Ir aufweist,
c) Zuführen von H₂ und CO,
d) Erwärmen des Reaktionsgemisches, wobei das Olefin zu einem Aldehyd umgesetzt wird.

Hierbei können die Verfahrensschritte a) bis d) in beliebiger Reihenfolge erfolgen.

Es kann hierbei auch ein Überschuss an Liganden verwendet werden und nicht zwangläufig jeder Ligand liegt gebunden in Form eines Ligand-Metall-Komplexes vor, sondern ist als freier Ligand im Reaktionsgemisch enthalten.

Die Reaktion wird bei üblichen Bedingungen durchgeführt.

Bevorzugt sind eine Temperatur von 80 °C bis 160 °C und ein Druck von 1 bar bis 300 bar. Besonders bevorzugt sind eine Temperatur von 100 °C bis 160 °C und ein Druck von 15 bar bis 250 bar.

Die Edukte für die Hydroformylierung gemäß dem Verfahren der Erfindung sind Olefine oder Gemische von Olefinen, insbesondere Monoolefine mit 2 bis 24, bevorzugt 3 bis 16, besonders bevorzugt 3 bis 12 Kohlenstoffatomen mit end- oder innenständigen C-C-Doppelbindungen, wie z.B. 1-Propen, 1- oder 2-Penten, 2-Methyl-1-buten, 2-Methyl-2-buten, 3-Methyl-1-buten, 1-, 2- oder 3,-Hexen, das bei der Dimerisierung von Propen anfallende C₆-Olefingemisch (Dipropen), Heptene, 2- oder 3-Methyl-1-hexene, Octene, 2-Methylheptene, 3-Methylheptene, 5-Methyl-2-hepten, 6-Methyl-2-hepten, 2-Ethyl-1-hexen, das bei der Dimerisierung von Butenen anfallende C₈-Olefingemisch (Dibuten), Nonene, 2- oder 3-Methyloctene, das bei der Trimerisierung von Propen anfallende C₉-Olefingemisch (Tripropen), Decene, 2-Ethyl-1-octen, Dodecene, das bei der Tetramerisierung oder der Trimerisierung von Butenen anfallende C₁₂-Olefingemisch (Tetrapropen oder Tributen), Tetradecene, Hexadecene, das bei der Tetramerisierung von Butenen anfallende C₁₆-Olefingemisch (Tetrabutan) sowie durch Cooligomerisierung von Olefinen mit unterschiedlicher Anzahl von Kohlenstoffatomen (bevorzugt 2 bis 4) hergestellte Olefingemische.

Im Folgenden wird die Erfindung anhand von Ausführungsbeispielen und einer Figur näher erläutert.

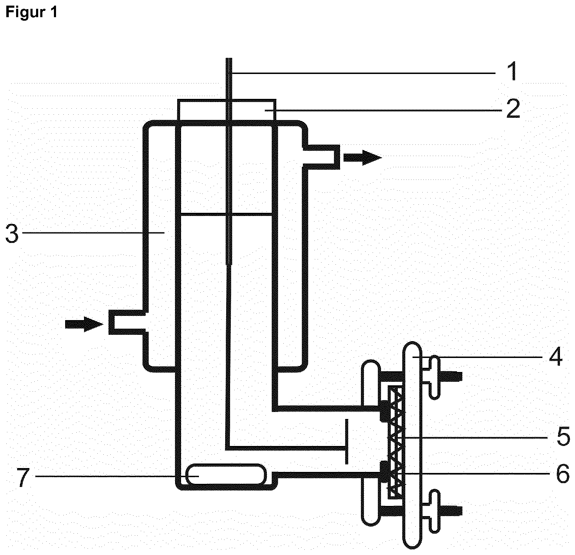

Die Figur 1 zeigt eine Reaktionsapparatur, in welcher die Kupplungsreaktion zu den entsprechenden unsymmetrischen Biarylen durchgeführt werden kann. Die Apparatur umfasst eine Nickelkathode (1) und eine Anode aus Bor-dotiertem Diamant (BDD) auf Silizium (5). Die Apparatur kann mit Hilfe des Kühlmantels (3) gekühlt werden. Die Pfeile deuten hierbei die Durchflussrichtung des Kühlwassers an. Der Reaktionsraum ist mit einem Teflonstopfen (2) verschlossen. Das Reaktionsgemisch wird durch ein Magnetrührstäbchen (7) durchmischt. Auf der anodischen Seite wird die Apparatur durch Schraubzwingen (4) und Dichtungen (6) verschlossen.

### Analytik

### Chromatographie

Die präparativen flüssigkeitschromatographischen Trennungen via "Flashchromatographie" wurden mit einem Maximaldruck von 1.6 bar an Kieselgel 60 M (0.040-0.063 mm) der Firma Macherey-Nagel GmbH & Co, Düren durchgeführt. Die Trennungen ohne Druckbeaufschlagung wurden an Kieselgel Geduran Si 60 (0.063-0.200 mm) der Firma Merck KGaA, Darmstadt durchgeführt. Die als Eluentien verwendeten Lösungsmittel (Essigsäureethylester (technisch), Cyclohexan (technisch)) wurden zuvor destillativ am Rotationsverdampfer gereinigt.

Zur Dünnschichtchromatographie (DC) wurden PSC-Fertigplatten Kieselgel 60 F254 der Firma Merck KGaA, Darmstadt verwendet. Die Rf-Werte sind in Abhängigkeit vom verwendeten Laufmittelgemisch angegeben. Zur Anfärbung der DC-Platten wurde eine Cer-Molybdatophosphorsäure-Lösung als Tauchreagenz verwendet. Cer-Molybdatophosphorsäure-Reagenz: 5.6 g Molybdatophosphorsäure, 2.2 g Cer(IV)-sulfat-Tetrahydrat und 13.3g konzentrierte Schwefelsäure auf 200 mL Wasser.

### Gaschromatographie (GC/GCMS)

Die gaschromatographischen Untersuchungen (GC) von Produktgemischen und Reinsubstanzen erfolgte mit Hilfe des Gaschromatographen GC-2010 der Firma Shimadzu, Japan. Es wird an einer Quarzkapillarsäule HP-5 der Firma Agilent Technologies, USA (Länge: 30 m; Innendurchmesser: 0.25 mm; Filmdicke der kovalent gebundenen stationären Phase: 0.25 µm; Trägergas: Wasserstoff; Injektortemperatur: 250 °C; Detektortemperatur: 310 °C; Programm:

Methode "hart": 50 °C Starttemperatur für 1 min, Heizrate: 15 °C/min, 290 °C Endtemperatur für 8 min) gemessen. Gaschromatographische Massenspektren (GCMS) von Produktgemischen und Reinsubstanzen wurden mit Hilfe des Gaschromatographen GC-2010 kombiniert mit dem Massendetektor GCMS-QP2010 der Firma Shimadzu, Japan aufgenommen. Es wird an einer Quarzkapillarsäule HP-1 der Firma Agilent Technologies, USA (Länge: 30 m; Innendurchmesser: 0.25 mm; Filmdicke der kovalent gebundenen stationären Phase: 0.25 µm; Trägergas: Wasserstoff; Injektortemperatur: 250 °C; Detektortemperatur: 310 °C; Programm: Methode "hart": 50 °C Starttemperatur für 1 min, Heizrate: 15 °C/min, 290 °C Endtemperatur für 8 min; GCMS: Temperatur der lonenquelle: 200 °C) gemessen.

### Schmelzpunkte

Schmelzpunkte wurden mit Hilfe des Schmelzpunktbestimmungsgerätes SG 2000 der Firma HW5, Mainz gemessen und sind unkorrigiert.

### Elementaranalyse

Die Elementaranalysen wurden in der analytischen Abteilung des Institutes für Organische Chemie der Johannes Gutenberg-Universität Mainz an einem Vario EL Cube der Firma Foss-Heraeus, Haunau angefertigt.

### Massenspektrometrie

Alle Elektrosprayionisation-Messungen (ESI+) wurden an einem QTof Ultima 3 der Firma Waters Micromasses, Milford, Massachusetts durchgeführt. EI-Massenspektren sowie die hochaufgelösten EI-Spektren wurden an einem Gerät des Typs MAT 95 XL Sektorfeldgerät der Firma ThermoFinnigan, Bremen, gemessen.

### NMR-Spektroskopie

Die NMR-spektroskopischen Untersuchungen wurden an Multikernresonanzspektrometern des Typs AC 300 oder AV II 400 der Firma Bruker, Analytische Messtechnik, Karlsruhe, durchgeführt. Als Lösungsmittel wurde CDCl3 verwendet. Die 1H- und 13C-Spektren wurden gemäß dem Restgehalt an nicht deuteriertem Lösungsmittel nach der NMR Solvent Data Chart der Fa. Cambridge Isotopes Laboratories, USA, kalibriert. Die Zuordnung der 1H- und 13C-Signale erfolgte teilweise mit Hilfe von H,H-COSY, H,H-NOESY, H,C-HSQC und H,C-HMBC-Spektren. Die chemischen Verschiebungen sind als δ-Werte in ppm angegeben. Für die Multiplizitäten der NMR-Signale wurden folgende Abkürzungen verwendet: s (Singulett), bs (breites Singulett), d (Dublett), t (Triplett), q (Quartett), m (Multiplett), dd (Dublett von Dublett), dt (Dublett von Triplett), tq (Triplett von Quartett). Alle Kopplungskonstanten J wurden mit der Anzahl der eingeschlossenen Bindungen in Hertz (Hz) angegeben. Die bei der Signalzuordnung angegebene Nummerierung entspricht der in den Formelschemata angegebenen Bezifferung, die nicht mit der IUPAC-Nomenklatur übereinstimmen muss.

### Allgemeine Arbeitsvorschriften

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).

### Synthese der Chlorophosphite

6-Chlorodibenzo[*d*,*f*][1,3,2]dioxaphosphepin wurde nach DE 10 2008 043 584 und 2-Chloro-4,4,5,5-tetraphenyl-1,3,2-dioxaphospholan nach DE 10 2006 058 682 hergestellt.

### Synthese der unsymmetrischen Biaryle

Die unsymmetrischen Biaryle wurden durch ein elektrochemisches Verfahren durch Kupplung von zwei Phenolen bzw. von einem Naphthol mit einem Phenol, welche sich in ihrem Oxidationspotential unterscheiden, hergestellt. Siehe hierzu auch B. Elsler, D. Schollmeyer, K. M. Dyballa, R. Franke, S. R. Waldvogel, "Metall- und reagensfreie hochselektive anodische Kreuzkupplung von Phenolen", Angew. Chem., 2014, DOI: 10.1002/ange.201400627

### Allgemeine Arbeitsvorschrift:

Die Kupplungsreaktion wurde in einer Apparatur durchgeführt, wie sie in Figur 1 dargestellt ist. 5 mmol des ersten Phenols mit einem Oxidationspotential *E_{Ox}1* werden mit 15 mmol des zweiten Phenols mit einem Oxidationspotential *E_{Ox}2* in den in der nachfolgenden Tabelle 1 angegebenen Mengen in 1,1,1,3,3,3-Hexafluorisopropanol (HFIP) und MeOH oder in Ameisensäure und MeOH gelöst. Die Elektrolyse erfolgt galvanostatisch. Der Außenmantel der Elektrolysezelle wird über einen Thermostaten auf etwa 10 °C temperiert, während die Reaktionsmischung gerührt und auf 50 °C mit Hilfe eines Sandbades erhitzt wird. Nach Ende der Elektrolyse wird der Zellinhalt mit Toluol in einen 50 mL Rundhalskolben überführt und das Lösungsmittel unter vermindertem Druck am Rotationsverdampfer bei 50 °C, 200-70 mbar entfernt. Nicht umgesetztes Edukt wird mittels Kurzwegdestillation zurückerhalten (100 °C, 10⁻³ mbar).

**Elektrodenmaterial**

| | |
|---|---|
| Anode: | Bor-dotierter Diamant (BDD) auf Si |
| Kathode: | Ni-Netz |

**Elektrolysebedingungen:**

| | |
|---|---|
| Temperatur [T]: | 50 °C |
| Stromstärke [I]: | 15 mA |
| Stromdichte [j]: | 2.8 mA/cm² |
| Ladungsmenge [Q]: | 2 F/mol Unterschusskomponente |
| Klemmspannung [Uₘₐₓ]: | 3-5 V |

Die Synthese der Biaryle erfolgte gemäß der oben beschriebenen allgemeinen Arbeitsvorschrift, und in einer Reaktionsapparatur, wie sie in Figur 1 dargestellt ist.

### 2,2'-Dihydroxy-4',5-dimethyl-5'-(methylethyl)-3-methoxybiphenyl

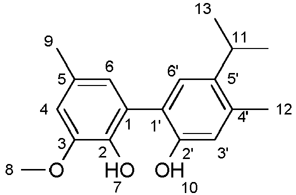

Es wurden 0.69 g (5 mmol, 1.0 Äquiv.) 4-Methylguajacol und 2.28 g (15 mmol, 3.0 Äquiv.) 3-Methyl-4-(methylethyl)phenol in 33 mL HFIP gelöst, 0.68 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 9:1 (Cyclohexan:Essigsäureethylester) aufgereinigt und das Produkt als farbloser Feststoff erhalten.
Ausbeute: 716 mg (50%, 2.5 mmol)
GC (Methode *hart,* HP-5): t_{R}= 14.87 min
R_{f}(CH:EE= 4:1)= 0.43
mₚ= 126.8°C (aus CH umkristallisiert)
¹H-NMR (600 MHz, DMSO) δ= 1.17-1.12 (m, 6H, 13-H), 2.24 (m, 6H, 9-H/12-H), 3.01 (dt, 1H, 11-H), 3.79 (s, 3H, 8-H), 6.55 (s, 1H, 6-H), 6.66 (d, 1H, 6'-H), 6.73 (d, 1H, 4-H), 6.96 (s, 1H, 3'-H), 8.16 (s, 1H, 7-H), 8.84 (s, 1H, 10-H);
Kopplungen: ⁴J_{4-H}, _{6-H}= 2.2 Hz, ⁴J_{6'-H, 11-H}= 2.9 Hz, ³J_{11-H, 13-H}= 6.8 Hz;
¹³C-NMR (151 MHz, DMSO) δ= 18.73, 20.80 (C-9/C-12), 23.54 (C-13), 28.10 (C-11), 55.78 (C-8), 111.23 (C-4), 117.34 (C-6'), 123.42 (C-1'), 123.49 (C-6), 126.43 (C-1), 127.36 (C-5), 127.49 (C-3'), 134.40 (C-5'), 136.62 (C-4'), 141.12 (C-2), 147.65 (C-3), 151.69 (C-2').
HRMS für C₁₈H₂₂O₃ (ESI+) [M+Na⁺]: ber: 309.1467, gef.: 309.1457
MS (EI, GCMS): m/z(%): 286 (50) [M]^{+·}, 271 (100) [M-CH₃^{·}]⁺, 244 (22) [M-C₃H₆^{·}]⁺. Elementaranalyse für C₁₈H₂₂O_{3:} ber: C: 75.50%, H: 7.74%, gef.: C: 75.01%, H: 7.70%.

### 2,2'-Dihydroxy-5,5'-dimethyl-3-methoxybiphenyl

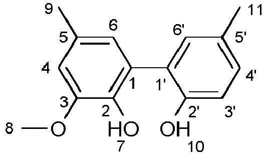

Es wurden 1.66 g (12 mmol, 1.0 Äquiv.) 4-Methylguajacol und 3.91 g (36 mmol, 3.0 Äquiv.) 4-Methylphenol in 65 mL HFIP und 14 mL MeOH gelöst, 1.63 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und das Produkt als farbloser Feststoff erhalten.
Ausbeute: 440 mg (36%, 1.8 mmol)
GC (Methode *hart,* HP-5): t_{R}= 13.56 min
R_{f}(CH:EE= 4:1)= 0.38
mₚ= 162.0 °C (aus CH umkristallisiert)
¹H-NMR (400 MHz, DMSO) δ= 2.18 (s, 3H, 9-H/11-H), 2.21 (s, 3H, 9-H/11-H), 3.76 (s, 3H, 8-H), 6.53 (s, 1H, 6-H), 6.71 (s, 1H, 4-H), 6.75 (d, 1H, 3'-H), 6.86-6.94 (m, 2H, 4'-H/6'-H), 8.53 (bs, 1H, 7-H/12-H);
Kopplungen: ³J_{3'-H, 4'-H}= 8.4 Hz;
¹³C-NMR (101 MHz, DMSO) δ= 20.21, 20.77 (C-9/C-11), 55.79 (C-8), 111.36 (C-4), 115.69 (C-3'), 123.50 (C-6), 125.72 (C-1'), 126.16 (C-1), 127.20 (C-5), 127.30 (C-5'), 128.50 (C-6'), 131.83 (C-4'), 141.20 (C-2), 147.61 (C-3), 152.11 (C-2').
HRMS für C₁₅H₁₆O₃ (ESI+) [M+Na⁺]: ber: 267.0997, gef.: 267.0999
MS (EI, GCMS): m/z(%): 244 (100) [M]^{+·}, 229 (64) [M-CH₃^{·}]⁺.
Elementaranalyse für C₁₅H₁₆O_{3:} ber: C: 73.75%, H: 6.60%, gef.: C: 73.81%, H: 6.54%.

### 2,2'-Dihydroxy-3-methoxy-3',5,5'-trimethyl-biphenyl

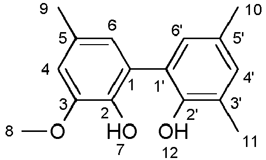

Es wurden 0.70 g (6 mmol, 1.0 Äquiv.) 4-Methylguajacol und 2.08 g (17 mmol, 3.0 Äquiv.) 2,4-Dimethylphenol in 27 mL HFIP und 6 mL MeOH gelöst, 0.68 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 9:1 (Cyclohexan:Essigsäureethylester) aufgereinigt und das Produkt als leichtgelber Feststoff erhalten.
Ausbeute: 663 mg (45%, 2.5 mmol)
GC (Methode *hart,* HP-5): t_{R}= 13.97 min
R_{f}(CH:EE= 4:1)= 0.29
mₚ= 119.7 °C (aus DCM/CH umkristallisiert)
¹H-NMR (400 MHz, CDCl₃) δ= 2.34 (s, 3H, 10-H), 2.35 (s, 3H, 11-H), 2.38 (s, 3H, 9-H), 3.94 (s, 3H, 8-H), 6.16 (s, 1H, 12-H), 6.20 (s, 1H, 7-H), 6.76 (d, 1H, 4-H), 6.78 (d, 1H, 6-H), 6.98 (d, 1H, 6'-H), 7.03 (d, 1H, 4'-H);
Kopplungen: ⁴J_{4-H, 6-H}= 1.7 Hz, ⁴J_{4'-H, 6'-H}= 2.1 Hz;
¹³C-NMR (101 MHz, CDCl₃) δ= 16.51 (C-9), 20.54 (C-10), 21.20 (C-11), 56.12 (C-8), 110.92 (C-4), 123.95 (C-6), 124.13 (C-1), 124.64 (C-1'), 126.18 (C-3'), 128.82 (C-6'), 129.59 (C-5'), 130.40 (C-5), 131.40 (C-4'), 139.46 (C-2), 146.35 (C-3), 149.42 (C-2').
HRMS für C₁₈H₁₆O₃ (ESI+) [M+Na⁺]: ber: 281.1154, gef.: 281.1152
MS (EI, GCMS): m/z(%): 242 (100) [M]^{+·}, 227 (38) [M-CH₃^{·}]⁺.
Elementaranalyse für C₁₆H₁₈O_{3:} ber: C: 68.31%, H: 6.45%, gef.: C: 68.29%, H: 6.40%.

### 2,2'-Dihydroxy-3-methoxy-5-methyl-4'-(dimethylethyl)biphenyl

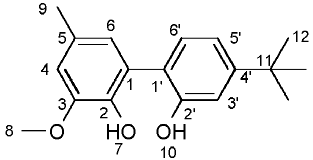

Es wurden 0.69 g (5 mmol, 1.0 Äquiv.) 4-Methylguajacol und 2.25 g (15 mmol, 3.0 Äquiv.) 3-tert-Butylphenol in 33 mL HFIP gelöst, 0.68 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und das Produkt als farbloser Feststoff erhalten.
Ausbeute: 808 mg (63%, 3.1 mmol)
GC (Methode *hart,* HP-5): t_{R}= 13.97 min
R_{f}(CH:EE= 4:1)= 0.29
mₚ= 160.3 °C (aus DCM/CH umkristallisiert)
¹H-NMR (400 MHz, CDCl₃) δ= 1.37 (s, 9H, 12-H), 2.36 (s, 3H, 9-H), 3.94 (s, 3H, 8-H), 6.25 (s, 1H, 7-H), 6.48 (s, 1H, 10-H), 6.75 (d, 1H, 6-H), 6.79 (d, 1H, 4-H), 7.08 (dd, 1H, 5'-H), 7.12 (d, 1H, 3'-H), 7.27 (d, 1H, 6'-H);
Kopplungen: ⁴J_{4-H, 6-H}= 1.7 Hz; ³J_{5'-H, 6'-H}= 8.0 Hz, ⁴J_{3'-H, 5'-H}= 1.7 Hz;
¹³C-NMR (101 MHz, CDCl₃) δ= 21.24 (C-9), 31.31 (C-12), 34.58 (C-11), 56.15 (C-8), 110.79 (C-4), 114.94 (C-3'), 118.30 (C-5'), 122.37 (C-1'), 123.88 (C-1), 123.94 (C-6), 130.45 (C-6'), 130.53 (C-4'), 139.24 (C-5), 146.32 (C-3), 152.91 (C-2'), 153.13 (C-2).
HRMS für C₁₅H₁₆O₄ (ESI+) [M+Na⁺]: ber: 309.1467, gef.: 309.1466
MS (EI, GCMS): m/z(%): 242 (100) [M]^{+·}, 227 (38) [M-CH₃^{·}]⁺.
Elementaranalyse für C₁₈H₂₂O_{3:} ber: 75.50%, H: 7.74%, gef.: C: 75.41%, H: 7.72%.

### 2,2'-Dihydroxy-3-methoxy-4'-5,5'-trimethylbiphenyl

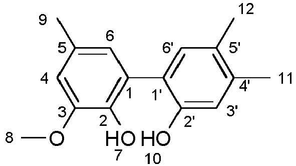

Es wurden 0.69 g (5 mmol, 1.0 Äquiv.) 4-Methylguajacol und 1.83 g (15 mmol, 3.0 Äquiv.) 3,4-Dimethylphenol in 27 mL HFIP und 6 mL MeOH gelöst, 0.68 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 9:1 (Cyclohexan:Essigsäureethylester) aufgereinigt und das Produkt als farbloser Feststoff erhalten.
Ausbeute: 688 mg (52%, 2.6 mmol)
GC (Methode *hart,* HP-5): t_{R}= 14.52 min
R_{f}(CH:EE= 4:1)= 0.29
mₚ= 152.3 °C (aus DCM/CH umkristallisiert)
¹H-NMR (400 MHz, CDCl₃) δ= 12.25 (s, 3H, 11-H), 2.28 (s, 3H, 12-H), 2.36 (s, 3H, 9-H), 3.93 (s, 3H, 8-H), 6.19 (s, 1H, 7-H), 6.25 (s, 1H, 10-H), 6.73 (d, 1H, 4-H), 6.76 (s, 1H, 6-H), 6.88 (s, 1H, 3'-H), 7.08 (s, 1H, 6'-H);
Kopplungen: ⁴J_{4-H, 6-H}= 1.7 Hz;
¹³C-NMR (101 MHz, CDCl₃) δ= 18.89 (C-11), 19.60 (C-12), 21.24 (C-9), 56.14 (C-8), 110.74 (C-4), 118.93 (C-3'), 122.54 (C-1), 123.82 (C-6), 123.97 (C-1'), 129.03 (C-5), 130.46 (C-4'), 131.69 (C-6'), 137.94 (C-5'), 139.26 (C-2), 146.31 (C-3), 151.36 (C-2').
HRMS für C₁₆H₁₈O₃ (ESI+) [M+Na⁺]: ber: 281.1154, gef.: 281.1157
MS (EI, GCMS): m/z(%): 258 (100) [M]^{+·}, 243 (10) [M-CH₃^{·}]⁺.
Elementaranalyse für C₁₆H₁₈O_{3:} ber: 74.39%, H: 7.02%, gef.: C: 74.32%, H: 7.20%

### 6,6'-((4'-(tert-Butyl)-3-methoxy-5-methyl-[1,1'-biphenyl]-2,2'-diyl)bis(oxy))didibenzo[d,f][1,3,2]dioxaphosphepin.

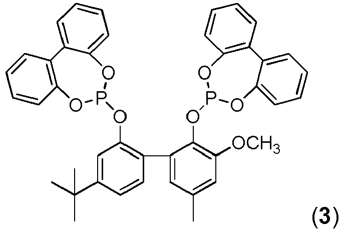

Eine Lösung von 4'-(*tert*-Butyl)-3-methoxy-5-methyl-[1,1'-biphenyl]-2,2'-diol (0,489 g; 1,708 mmol) in Toluol (12 ml) wurde mit Pyridin (0,389 g; 3,844 mmol) versetzt und die erhaltene Mischung bei 3 °C tropfenweise zu einer Lösung von 6-Chlorodibenzo[*d*,*f*][1,3,2]dioxaphosphepin (0,942 g; 3,758 mmol) in Toluol (12 ml) gegeben. Die Reaktionsmischung wurde über Nacht gerührt und dann filtriert. Das Filtrat wurde im Vakuum bis zur Trockne eingeengt und der erhaltene Feststoff bei 50 °C / 0,1 mbar getrocknet. Die zurückgebliebene Substanz wurde durch Säulenchromatografie gereinigt (Hexan/Toluol, 1:2, R*_{f}* = 0,3). Ausbeute: 0,738 g (1,032 mmol; 58 %).
Elementaranalyse (ber. für C₄₂H₃₆O₇P₂ = 714,69 g/ mol) C 70,59 (70,58); H 5,28 (5,08); P 8,85 (8,67) %.
³¹P-NMR (CD₂Cl₂): 144,3 (d, *J*_{PP} = 9,1 Hz); 148,1 (d, *J*_{PP} = 9,1 Hz) ppm.
¹H-NMR (CD₂Cl₂): 1,51 (m, 9 H); 2,45 (m, 3 H); 4,06 (s, 3 H); 6,80-6,87 (m, 3 H, Hₐᵣₒₘ); 6,98-7,03 (m, 2 H, Hₐᵣₒₘ); 7,03-7,05 (m, 1 H, Hₐᵣₒₘ); 7,28-7,35 (m, 8 H, Hₐᵣₒₘ); 7,35-7,38 (m, 1 H, Hₐᵣₒₘ); 7,38-7,43 (m, 2 H, Hₐᵣₒₘ); 7,46-7,54 (m, 4 H, Hₐᵣₒₘ) ppm.
¹³C-NMR (CD₂Cl₂): 21,5; 57,0; 113,8; 118,4 (d, *J*_{CP}= 10,1 Hz); 121,8; 122,5 (d, *J*_{CP}= 14,1 Hz); 124,9; 125,5 (d, *J*_{CP}= 17,3 Hz); 127,9; 128,7; 129,4; 129,4 (d, *J*_{CP}= 16,8 Hz); 130,1 (d, *J*_{CP}= 16,1 Hz); 131,3; 131,5; 131,6; 132,0; 134,4; 138,0; 149,5 (d, *J*_{CP}= 4,8 Hz); 149,7 (d, *J*_{CP}= 4,4 Hz); 149,8 (d, *J*_{CP}= 7,0 Hz); 151,2 (d, *J*_{CP}= 3,2 Hz); 153,4 ppm.

### 6,6'-((5'-Isopropyl-3-methoxy-4',5-dimethyl-[1,1'-biphenyl]-2,2'-diyl)bis(oxy))didibenzo[d,f][1,3,2]dioxaphosphepin.

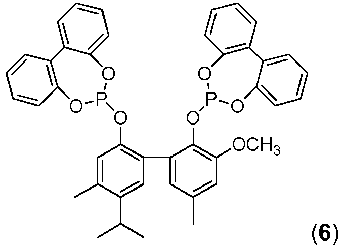

Eine Lösung von 5'-Isopropyl-3-methoxy-4',5-dimethyl-[1,1'-biphenyl]-2,2'-diol (0,485 g; 1,692 mmol) in Toluol (12 ml) wurde mit Pyridin (0,385 g; 3,807 mmol) versetzt und die erhaltene Mischung tropfenweise zu einer auf 3 °C gekühlten Lösung von 6-Chlorodibenzo[*d*,*f*][1,3,2]dioxaphosphepin (0,933 g; 3,722 mmol) in Toluol (12 ml) gegeben. Die Reaktionsmischung wurde über Nacht gerührt und dann filtriert. Das Filtrat wurde im Vakuum bis zur Trockne eingedampft und der erhaltene Feststoff bei 50 °C /0,1 mbar getrocknet. Die zurückgebliebene Substanz wurde durch Säulenchromatografie gereinigt (Hexan/Toluol, 1:4, R*_{f}* = 0,4). Ausbeute: 0,725 g (1,015 mmol; 60 %).
Elementaranalyse (ber. für C₄₂H₃₆O₇P₂ = 714,69 g/ mol) C 70,77 (70,58); H 5,28 (4,98); P 8,85 (8,86) %.
³¹P-NMR (CD₂Cl₂): 145,4 (d, *J*_{PP} = 7,2 Hz); 148,2 (d, *J*_{PP} = 7,2 Hz) ppm.
¹H-NMR (CD₂Cl₂): 1,24 (s, 3 H); 1,26 (s, 3 H); 2,43 (m, 3 H); 2,51 (m, 3 H); 3,22 (m, 1 H); 4,03 (s, 3 H); 6,72-6,83 (m, br, 3 H, Hₐᵣₒₘ); 6,95-6,99 (m, 2 H, Hₐᵣₒₘ); 6,99-7,02 (m, 1 H, Hₐᵣₒₘ); 7,13 (m, 1 H, Hₐᵣₒₘ); 7,22-7,33 (m, 9 H, Hₐᵣₒₘ); 7,42-7,50 (m, 4 H, Hₐᵣₒₘ) ppm.
¹³C-NMR (CD₂Cl₂): 19,3; 21,5; 23,5; 29,4; 57,0; 113,8; 122,6 (d, *J*_{CP}= 14,8 Hz); 124,8; 125,5 (d, *J*_{CP}= 18,7 Hz); 128,3; 128,8; 129,4 (d, *J*_{CP}= 14,5 Hz); 130,0 (d, *J*_{CP}= 16,3 Hz); 131,5; 131,6; 134,3; 136,8; 137,9 (d, *J*_{CP}= 6,3 Hz); 143,4; 147,5 (d, *J*_{CP}= 8,2 Hz); 149,6 (d, *J*_{CP}= 5,0 Hz); 149,7 (d, *J*_{CP}= 4,5 Hz); 151,0 (d, *J*_{CP}= 2,8 Hz) ppm.

### 6,6'-((3-Methoxy-5,5'-dimethyl-[1,1'-biphenyl]-2,2'-diyl)bis(oxy))didibenzo[d,f][1,3,2]dioxaphosphepin.

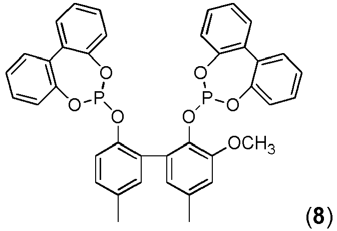

Eine Lösung von 3-Methoxy-5,5'-dimethyl-[1,1'-biphenyl]-2,2'-diol (0,419 g; 1,714 mmol) in Toluol (12 ml) wurde mit Pyridin (0,390 g; 3,857 mmol) versetzt und die erhaltene Mischung bei 3 °C tropfenweise zu einer Lösung von 6-Chlorodibenzo[*d*,*f*][1,3,2]dioxaphosphepin (0,945 g; 3,771 mmol) in Toluol (12 ml) gegeben. Die Reaktionsmischung wurde über Nacht gerührt und dann filtriert. Das Filtrat wurde im Vakkum bis zur Trockne eingeengt und der erhaltene Feststoff bei 50 °C getrocknet. Die Substanz wurde in heißem Acetonitril gelöst (25 ml). Kristallisation erfolgt nach Abkühlen der Lösung und Einengen im Vakuum auf die Hälfte des Volumens. Ausbeute: 0,205 g (0,030 mmol; 18 %).
Elementaranalyse (ber. für C₃₉H₃₀O₇P₂ = 672,61 g/ mol) C 69,58 (69,64); H 4,30 (4,49); P 9,25 (9,21) %.
³¹P-NMR (CD₂Cl₂): 145,6 (d, *J*_{PP} = 8,4 Hz); 148,2 (d, *J*_{PP} = 8,4 Hz) ppm.
¹H-NMR (CD₂Cl₂): 42 (m, 6 H); 4,05 (s, 3 H); 6,75-6,82 (m, 3 H, Hₐᵣₒₘ); 6,95-7,02 (m, 3 H, Hₐᵣₒₘ); 7,17 (m, 1 H, Hₐᵣₒₘ); 7,24-7,36 (m, 10 H, Hₐᵣₒₘ); 7,42-7,50 (m, 4 H, Hₐᵣₒₘ) ppm.
¹³C-NMR (CD₂Cl₂): 20,9; 21,4; 57,0; 113,9; 120,9 (d, *J*_{CP}= 10,3 Hz); 122,6 (d, *J*_{CP}= 11,6 Hz); 124,7; 125,5 (d, *J*_{CP}= 19,2 Hz); 129,4 (d, *J*_{CP}= 9,9 Hz); 129,8; 129,9; 130,1; 130,5 (d, *J*_{CP}= 3,3 Hz); 131,2 (d, *J*_{CP}= 3,0 Hz); 131,5 (m); 133,0; 134,3 (d, *J*_{CP}= 4,6 Hz); 147,8 (d, *J*_{CP}= 8,4 Hz); 149,4 (d, *J*_{CP}= 5,0 Hz); 149,7 (d, *J*_{CP}= 4,6 Hz); 151,0 (d, *J*_{CP}= 2,9 Hz) ppm.

### 6,6'-((3-Methoxy-3',5,5'-trimethyl-[1,1'-biphenyl]-2,2'-diyl)bis(oxy))didibenzo[d,f][1,3,2]dioxaphosphepin.

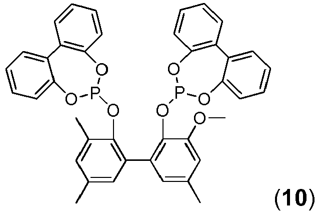

Eine Lösung von 4',5-Dimethoxy-6'-methyl-[1,1'-biphenyl]-2,3'-diol (0,361 g; 2,439 mmol) in Toluol (8 ml) wurde mit Triethylamin (0,444 g; 4,385 mmol) versetzt und auf 0 °C gekühlt. Zu dieser Mischung wurde eine Lösung von 6-Chlorodibenzo[*d*,*f*][1,3,2]dioxaphosphepin (0,57 g; 3,419 mmol) in Toluol (13 ml) tropfenweise zugegeben. Die Reaktionsmischung wurde für 45 min bei 0 °C und über Nacht bei Raumtemperatur gerührt, über Kieselgel filtriert und das Filtrat im Vakuum wurde bis zur Trockne eingeengt. Ausbeute: 0,666 g (0,696 mmol; 69 %). Elementaranalyse (ber. für C₄₀H₃₂O₇P₂ = 686,16 g/ mol): C 69,75 (69,97); H 4,76 (4,70); P 8,80 (9,02) %.
³¹P-NMR (CD₂Cl₂): 142,4 (d, *J*_{PP}= 9,7 Hz); 144,8 (d, *J*_{PP}= 9,7 Hz); 146,7 (d, *J*_{PP}= 13,6 Hz); 148,4 (d, *J*_{PP}= 13,6 Hz) ppm. ¹H-NMR (CD₂Cl₂): 2,01; 2,37; 2,41; 2,42; 2,46; 2,50 (6s, 9 H); 3,90; 4,04 (2s, 3 H); 6,76-7,63 (m, 20 H) ppm.

### 6,6'-((3-Methoxy-4',5,5'-trimethyl-[1,1'-biphenyl]-2,2'-diyl)bis(oxy))didibenzo[d,f][1,3,2]dioxaphosphepin.

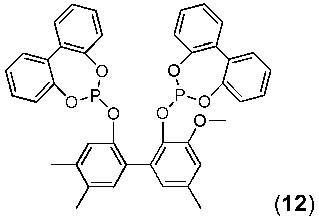

Eine Lösung von 3-Methoxy-4',5,5'-trimethyl-[1,1'-biphenyl]-2,2'-diol (0,334 g; 1,294 mmol) in Toluol (7 ml) wurde mit Triethylamin (0,410 g; 4,048 mmol) versetzt, auf 0 °C gekühlt und anschließend zu dieser Mischung eine Lösung von 6-Chlorodibenzo[*d*,*f*][1,3,2]dioxaphosphepin (0,791 g; 3,157 mmol) in Toluol (12 ml) tropfenweise zugegeben. Die Reaktionsmischung wurde für 45 min bei 0 °C und drei Tage bei Raumtemperatur gerührt. Die beige-gelbe Suspension wurde filtriert und der erhaltene Feststoff mit ein wenig eiskaltem Toluol gewaschen. Der Feststoff wurde in 40 ml Toluol aufgenommen. Die erhaltene Suspension wurde für 2 h bei 40 °C gerührt und dann warm filtriert. Das Filtrat wurde im Vakuum bis zur Trockne eingeengt und der Rückstand bei 0,1 mbar getrocknet. Ausbeute: 0,254 g (0,370 mmol; 29 %).
Elementaranalyse (ber. für C₄₀H₃₂O₇P₂ = 686,60 g/ mol) C 69,79 (69,97); H 4,81 (4,70); P 9,01 (9,02) %.
³¹P-NMR (CD₂Cl₂): 145,6 (d, *J*_{PP}= 8,4 Hz); 148,2 (d, *J*_{PP}= 8,4 Hz) ppm.
¹H-NMR (CD₂Cl₂): 2,31 (m, 3 H); 2,42 (m, 3 H); 2,43 (m, 3 H); 4,03 (s, 3 H); 6,75-6,81 (m, 3 H, Hₐᵣₒₘ); 6,94-6,98 (m, 2 H, Hₐᵣₒₘ); 7,00 (m, 1 H, Hₐᵣₒₘ); 7,12 (m, 2 H, Hₐᵣₒₘ); 7,23-7,35 (m, 8 H, Hₐᵣₒₘ); 7,42-7,50 (m, 4 H, Hₐᵣₒₘ) ppm.
¹³C-NMR (CD₂Cl₂): 19,2; 19,8; 21,4; 57,0; 113,7; 122,2 (d, *J*_{CP}= 10,1 Hz); 122,5 (d, *J*_{CP}= 13,1 Hz); 124,8; 125,3; 125,6; 125,6; 125,6; 127,9; (d, *J*_{CP}= 3,4 Hz); 128,6; 129,2 (d, *J*_{CP}= 15,5 Hz); 129,3; 129,8 (d, *J*_{CP}= 16,6 Hz); 131,2; 131,5 (m); 133,0; 133,2; 134,3; 137,9; 138,1; 147,8 (d, *J*_{CP}= 8,5 Hz); 149,5 (d, *J*_{CP}= 4,9 Hz); 149,7 (d, *J*_{CP}= 10,1 Hz); 150,9 (d, *J*_{CP}= 2,8 Hz) ppm.

### Arbeitsvorschrift für die Katalyseversuche

Die Hydroformylierung wurde in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Zur Minimierung eines Einflusses von Feuchtigkeit und Sauerstoff wurde das als Solvens benutzte Toluol mit Natrium-Ketyl getrocknet und unter Argon destilliert. Die als Substrat eingesetzten Substrate 1-Octen (Aldrich), *cis*/*trans-*2-Penten (Aldrich) und n-Octene (Oxeno GmbH, Octenisomerengemisch aus 1-Octen: ∼3 %; *cis*+*trans-*2-Octen; ∼49%; *cis*+*trans*-3-Octen: ∼29 %; *cis*+*trans*-Octen-4: ∼16 %; gerüstisomere Octene: ∼3 %) wurden mehrere Stunden über Natrium am Rückfluß erhitzt und unter Argon destilliert.

Für die Versuche wurden im Autoklaven unter Argonatmosphäre folgende Lösungen des Rhodiums in Form von [(acac)Rh(COD)] (acac=Acetylacetonat-Anion; COD=1,5-Cyclooctadien) (OMG AG & Co. KG, Hanau, DE) als Katalysatorvorstufe in Toluol eingefüllt: Für Versuche mit 100 ppm-m Rhodium 10 ml einer 4,31 millimolaren, für 40 ppm-m die gleiche Menge einer entsprechend verdünnte Lösung. Anschließend wurde die entsprechende Menge der in Toluol gelösten Phosphitverbindung, in der Regel 2 bis 5 Ligandäquivalente pro Rhodium, zugemischt. Durch Zugabe von weiterem Toluol (Die Gesamtmasse an Toluol wurde bestimmt für die GC-Analyse, s.u.) wurde das Anfangsvolumen der Katalysatorlösung auf a) 41,0 ml eingestellt bei beabsichtigter Zugabe von 15 ml des Olefins über die Druckpipette (1-Octen, n-Octenen und Versuche mit erhöhter Konzentration an 2-Penten), oder b) 51,9 ml eingestellt bei beabsichtigter Zugabe von 4,1 ml 2-Penten. Die jeweils eingebrachte Masse an Toluol wurde bestimmt. Einwaagen der Olefine: 1-Octen (10,62 g; 94,64 mmol), n-Octene (10,70 g; 95,35 mmol), 2-Penten 9,75 g; 139,00 mmol. Der Autoklav wurde bei einem Gesamtgasdruck (Synthesegas: Linde; H₂ (99,999%) : CO (99,997%) = 1:1) von a) 42 bar für einen Enddruck von 50 bar; b) 12 bar für den Enddruck von 20 bar und c) 7 bar für einen Enddruck von 10 bar unter Rühren (1500 U/min) auf die jeweils angegebenen Temperaturen aufgeheizt. Nach Erreichen der Reaktionstemperatur wurde der Synthesegasdruck auf a) 48,5 bar für einen Enddruck von 50 bar, b) 19,5 bar für einen Enddruck von 20 bar und c) 9,5 bar für einen Enddruck von 10 bar erhöht das jeweils in der Tabelle angegebene Olefin(-gemisch) mit einem in der Druckpipette eingestellten Überdruck von ca. 3 bar zugepresst. Die Reaktion wurde bei konstantem Druck von jeweils 50, 20 bzw. 10 bar (Nachdruckregler der Fa. Bronkhorst, NL) über 4h geführt. Der Autoklav wurde nach Ablauf der Reaktionszeit auf Zimmertemperatur abgekühlt, unter Rühren entspannt und mit Argon gespült. Jeweils 1 ml der Reaktionsmischungen wurden unmittelbar nach Abschalten des Rührers entnommen, mit 5 ml Pentan verdünnt und gaschromatographisch analysiert: HP 5890 Series II plus, PONA, 50 m x 0,2 mm x 0,5 µm, Die quantitative Bestimmung von Restolefin und Aldehyd erfolgte gegen das Lösungsmittel Toluol als internen Standard.

### Ergebnisse der Katalyseversuche

Solvens: Toluol
Ausb. = Ausbeute
Sel. = Selektivität
p = Druck in [bar]
T = Temperatur in [°C]
t = Zeit in [h]
[Rh] = Rhodiumkonzentration in [ppm]
L/Rh = Verhältnis von Ligand zum Rhodium

Als Vergleichsliganden wurden die Liganden **A** und **B** ausgewählt. Ihre Herstellung erfolgte nach DE 10 2006 058 682 A1.

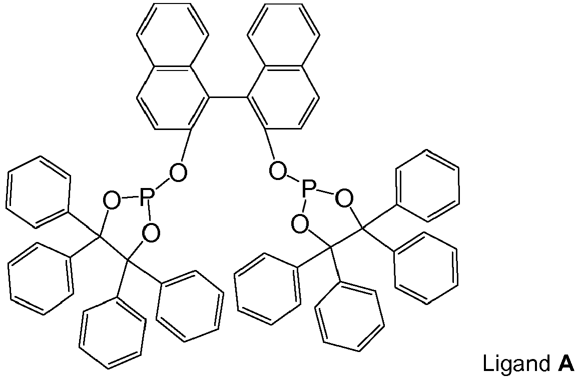

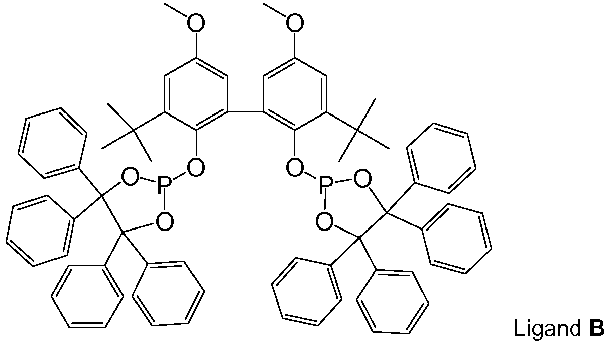

Die erfindungsgemäßen Verbindungen sind mit * gekennzeichnet.

**Tabelle 1: 1-Octen**

| **Ligand** | **p** | **T** | **t** | **[Rh]** | **L/Rh** | **Ausb.** | **Sel.** |
|---|---|---|---|---|---|---|---|
| | **(bar)** | **(°C)** | **(h)** | **(ppm)** | | **(%)** | **(%)** |
| **3*** | 50 | 100 | 4 | 40 | 2 | 90 | 90,0 |
| **6*** | 50 | 100 | 4 | 40 | 2 | 91 | 90,8 |
| **8*** | 50 | 100 | 4 | 40 | 2 | 93 | 90,2 |
| **10*** | 50 | 100 | 4 | 40 | 2 | 91 | 91,7 |
| **12*** | 50 | 100 | 4 | 40 | 2 | 93 | 90,4 |
| **A** | 50 | 100 | 4 | 40 | 2 | 89 | 83 |

Wie der Tabelle 1 zu entnehmen ist, zeichnen sich die Liganden in der Hydroformylierung von terminalen Olefinen, hier konkreter 1-Octen, durch sehr gute Ausbeuten aus. Alle erfindungsgemäßen Verbindungen haben eine Ausbeute, welche mindestens so gut ist, wie die des Vergleichsliganden **A.**

**Tabelle 2: 2-Penten**

| **Ligand** | **p** | **T** | **t** | **[Rh]** | **L/Rh** | **Ausb.** | **Sel.** |
|---|---|---|---|---|---|---|---|
| | **(bar)** | **(°C)** | **(h)** | **(ppm)** | | **(%)** | **(%)** |
| **3*** | 20 | 120 | 4 | 100 | 2 | 93 | 57,0 |
| **6*** | 20 | 120 | 4 | 100 | 2 | 89 | 57,6 |
| **8*** | 20 | 120 | 4 | 100 | 2 | 83 | 59,4 |
| **10*** | 20 | 120 | 4 | 100 | 2 | 90 | 63,0 |
| **12*** | 20 | 120 | 4 | 100 | 2 | 87 | 59,1 |
| **B** | 20 | 120 | 4 | 96 | 2 | 14 | 99 |

Mit dem Vergleichsliganden **B** konnte für 2-Penten zwar eine sehr gute Selektivität, 99 %, erzielt werden, jedoch ist die Ausbeute mit 14 % so gering, dass der Einsatz eines solchen Ligandens für ein großtechnisches Verfahren nur wenig interessant ist. Die Raum-Zeit-Ausbeuten mit diesem Liganden sind so schlecht, sodass dies aus ökonomischer Sicht gegen den Einsatz des Vergleichsliganden **B** spricht.

Die erfindungsgemäßen Verbindungen weißen alle eine annehmbare bis sehr gute Ausbeute in Kombination mit einer guten Selektivität auf.

Wie die Versuchsergebnisse zeigen, wird die gestellte Aufgabe durch die erfindungsgemäßen Verbindungen gelöst.

Es ist somit erstmalig gelungen, Bisphosphite zu generieren, die einen unsymmetrischen Biaryl-Zentral-Baustein, also ein unsymmetrisches Rückgrat, enthalten und gute bis sehr gute Hydroformylierungseigenschaften aufweisen. Dies konnte mit einer Vielzahl von Beispielen belegt werden. Solche konkreten Strukturen und derartige Liganden waren bis dato gänzlich unbekannt und nicht zugänglich.

Diese Bisphosphite weisen eine neuartige Asymmetrie auf. Das besondere hierbei ist die Asymmetrie innerhalb des Biaryl-Zentral-Bausteins, die zu unsymmetrischen Bisphosphiten führt. Diese unsymmetrischen Bisphosphite sind strukturell somit gänzlich von denen im Stand der Technik beschriebenen Bisphoshiten verschieden, bei denen unsymmetrische Bisphosphit-Liganden durch eine bestimmte Anordnung von symmetrischen Biaryl-Bausteinen generiert werden, indem sich beispielsweise die beiden Flügel-Bausteine unterscheiden, die einzelnen Bausteine (Zentral-Baustein und Flügel-Bausteine) an sich aber symmetrisch sind.

## Patentansprüche

1. Verbindung, welche eine der folgenden Strukturen aufweist:

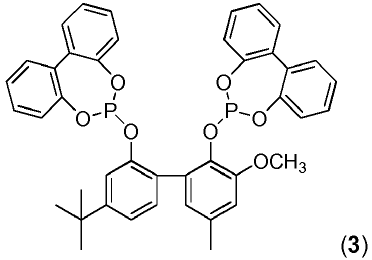

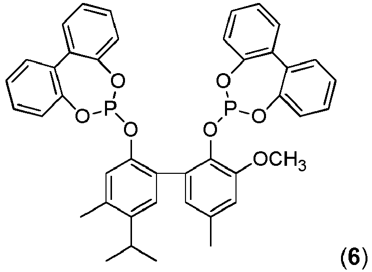

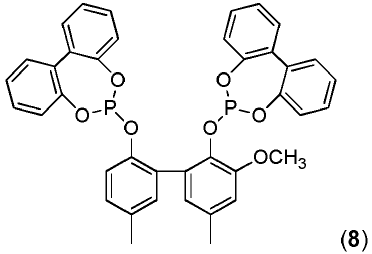

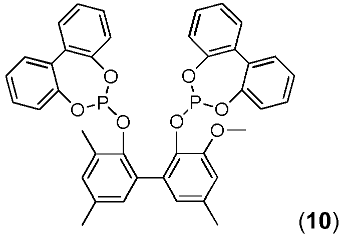

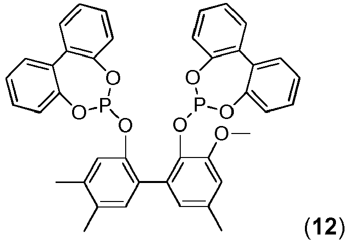

2. Komplex umfassend:
- eine Verbindung nach Anspruch 1,
- ein Metallatom ausgewählt aus: Rh, Ru, Co, Ir.

3. Verwendung einer Verbindung nach Anspruch 1,
zur Katalyse einer Hydroformylierungsreaktion.

4. Verfahren umfassend die Verfahrensschritte:
a) Vorlegen eines Olefins,
b) Zugabe eines Komplexes nach Anspruch 2,
oder einer Verbindung nach Anspruch 1 und einer Substanz, welche ein Metallatom ausgewählt aus: Rh, Ru, Co, Ir aufweist,
c) Zuführen von H₂ und CO,
d) Erwärmen des Reaktionsgemisches, wobei das Olefin zu einem Aldehyd umgesetzt wird.

## Claims

1. Compound having one of the following structures:

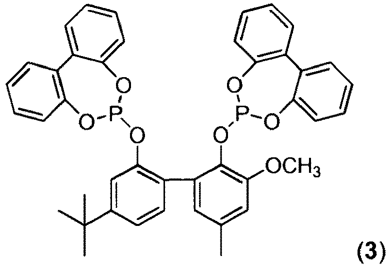

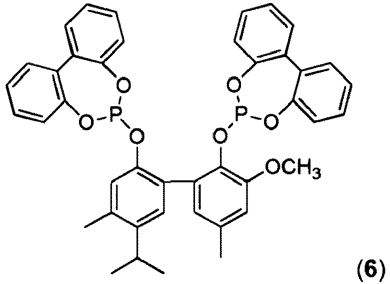

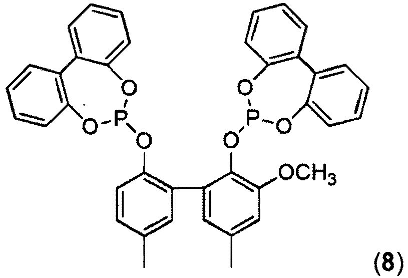

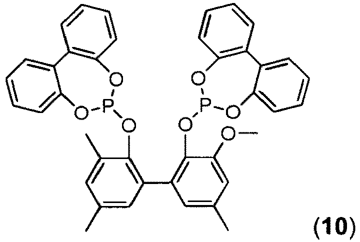

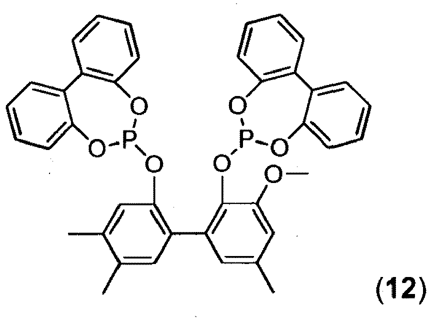

2. Complex comprising:
- a compound according to Claim 1,
- a metal atom selected from: Rh, Ru, Co, Ir.

3. Use of a compound according to Claim 1 for catalysis of a hydroformylation reaction.

4. Process comprising the process steps of:
a) initially charging an olefin,
b) adding a complex according to Claim 2
or a compound according to Claim 1 and a substance including a metal atom selected from: Rh, Ru, Co, Ir,
c) feeding in H₂ and CO,
d) heating the reaction mixture, with conversion of the olefin to an aldehyde.

## Revendications

1. Composé, qui présente une des structures suivantes :

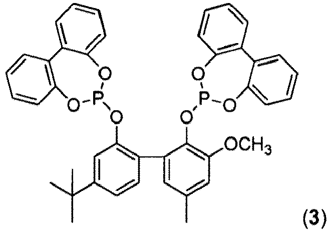

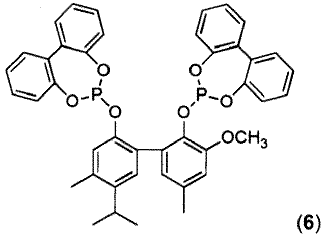

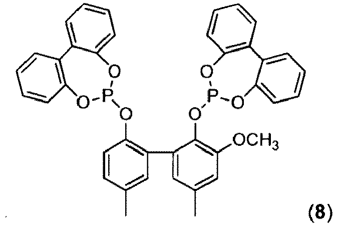

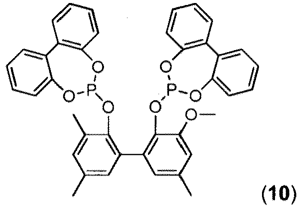

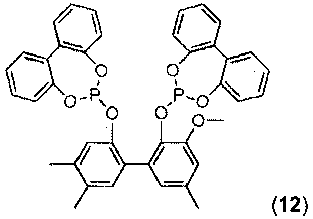

2. Complexe comprenant :
- un composé selon la revendication 1,
- un atome métallique choisi parmi : Rh, Ru, Co, Ir.

3. Utilisation d'un composé selon la revendication 1 pour la catalyse d'une réaction d'hydroformylation.

4. Procédé comprenant les étapes de procédé suivantes :
a) le chargement initial d'une oléfine,
b) l'ajout d'un complexe selon la revendication 2,
ou d'un composé selon la revendication 1 et d'une substance qui comprend un atome métallique choisi parmi : Rh, Ru, Co, Ir,
c) l'introduction d'H₂ et de CO,
d) le chauffage du mélange réactionnel, l'oléfine étant transformée en un aldéhyde.
